# EUROPEAN PATENT SPECIFICATION

(11) **EP 3 235 500 B1**
(45) Date of publication and mention of the grant of the patent: **06.05.2020**
(21) Application number: 16746863.6
(22) Date of filing: 04.02.2016
(51) Int. Cl.: A61K 35/28, A61K 35/51, A61K 35/32, A61K 9/08, A61P 19/02, C12N 5/0775

(54) **COMPOSITION FOR CHONDROCYTE DIFFERENTIATION INDUCTION OR CARTILAGE TISSUE REGENERATION, CONTAINING EXOSOMES EXTRACTED FROM STEM CELLS DIFFERENTIATING INTO CHONDROCYTES**
ZUSAMMENSETZUNG ZUR CHONDROZYTENDIFFERENZIERUNGSINDUKTION ODER KNORPELGEWEBEREGENERATION MIT AUS STAMMZELLEN EXTRAHIERTEN EXOSOMEN, DIE ZU CHONDROZYTEN DIFFERENZIEREN
COMPOSITION POUR INDUCTION DE DIFFÉRENCIATION DE CHONDROCYTE OU RÉGÉRÉRATION DE TISSU CARTILAGINEUX À BASE D'EXOSOMES EXTRAITS DE CELLULES SOUCHES SE DIFFÉRENCIANT EN CHONDROCYTES

(30) Priority: 04.02.2015 KR 20150017349; 03.02.2016 KR 20160013293
(43) Date of publication of application: 25.10.2017
(73) Proprietor: Exostemtech Co., Ltd., Sangrok-gu Ansan-si, Gyeonggi-do 15588 (KR)
(72) Inventor: CHO, Yong Woo, Seongnam-si Gyeonggi-do 13599 (KR); CHOI, Ji Suk, Ansan-si Gyeonggi-do 15521 (KR); WOO, Chang Hee, Gwangmyeong-si Gyeonggi-do 14206 (KR); CHOI, Young Chan, Ansan-si Gyeonggi-do 15588 (KR); JO, Dong Gyu, Gwacheon-si Gyeonggi-do 13834 (KR)
(74) Representative: Diehl & Partner GbR
(86) International application number: PCT/KR2016/001230
(87) International publication number: WO 2016/126122

(56) References cited:
- EP-A1- 1 077 253
- WO-A1-2014/013258
- WO-A2-2009/152084
- CN-A- 103 767 985
- JP-A- 2007 528 706
- US-A1- 2011 014 251
- REBECA BLAZQUEZ ET AL: "Immunomodulatory Potential of Human Adipose Mesenchymal Stem Cells Derived Exosomes on in vitro Stimulated T Cells", FRONTIERS IN IMMUNOLOGY, vol. 5, 4 October 2014 (2014-10-04), pages 1-9, XP055348822, CH ISSN: 1664-3224, DOI: 10.3389/fimmu.2014.00556
- MAUMUS, M. ET AL.: 'Mesenchymal Stem Cells in Regenerative Medicine applied to Rheumatic Diseases: Role of Secretome and Exosomes' BIOCHIMIE vol. 95, 2013, pages 2229 - 2234, XP028768026

## Description

### TECHNICAL FIELD

The present invention relates to relate to a composition for inducing chondrocyte differentiation or for regenerating cartilage tissue. The composition may include, as an active ingredient, an exosome derived from stem cells differentiating into chondrocytes. The present invention may include a medium composition for inducing chondrocyte differentiation; an injection preparation for regenerating cartilage tissue; and a pharmaceutical composition for treating cartilage disorders, all of which contain the composition for inducing chondrocyte differentiation or for regenerating cartilage tissue. The present invention may further include a method for treating cartilage disorders using the composition.

### BACKGROUND ART

Due to various characteristics of cartilage tissues, it is difficult to regenerate cartilage tissues by natural healing when a large area is damaged. Therefore, techniques have been developed for treating damaged cartilage tissues. For example, damaged cartilage tissues have been treated using surgical operations such as artificial joint replacement operations, chondroplasty of articular cartilage, microfracture technique, and the like. However, the aforesaid surgical techniques require forming incisions that problematically cause scars, and produce fibrocartilage having less durability. Accordingly, the therapeutic effect of the surgical techniques is not satisfactory even through complicated operations.

Therefore, injectable preparations using hydrogels have been developed, which can be delivered using simple surgical processes that do not require forming large incisions, and which produce fast therapeutic effects. For example, an injectable solution for intra-articular administration using an alkylene diamine crosslinked hydrogel of hyaluronic acid (see Korean Patent Publication No. 2013-0028012), a composition for repairing cartilage tissue containing collagen and hyaluronic acid (see Korean Patent Registration No. 1279812), a pharmaceutical formulation for the treatment of osteoarthritis containing clodronic acid and hyaluronic acid (see Korean Patent Publication No. 2008-0082657), and the like have been developed. However, although these methods can temporarily reduce pain, they insufficiently induce cartilage tissue regeneration. Thus, factors that effectively induce cell differentiation in order to regenerate cartilage tissues are needed.

Currently, methods in cell therapeutics for inducing the regeneration of cartilage tissue include transplanting cells cultured in vitro to a damaged tissue area. For example, therapeutic methods using autologous chondrocytes (see Korean Patent Publication No. 2013-0072983), stem cells derived from umbilical cords (see Korean Patent Publication No. 2013-0009651), and the like have been developed. However, therapeutic procedures to treat large damaged areas using autologous chondrocytes are not effective. This is because the procedures using autologous chondrocytes include harvesting cells from the patient, culturing the cells, and transplanting the cells into the patient. In addition, since the patient is subjected to at least two operations including a transplant surgery, the patient usually suffers from physical pain and an economic burden. Stem cell therapies generally include performing a cell treatment using stem cells such as umbilical cord blood, adipose tissue, synovial membranes, and muscular stem cells. However, the stem cell therapies may result in concerns such as differentiating into chondrocytes at a low rate after transplanting stem cells in the body, calcification of chondrocytes due to blood vessel infiltration, and apoptosis caused by gene expression associated with cell hypertrophy. The complications of stem cell therapies may occur because of the inconsistency in cell number and differentiation ability depending on the site of collection, and the change of cell phenotype due to cell dedifferentiation during in vitro culture.

As described above, conventional treatment techniques utilize methods in which chondrocytes or adult stem cells obtained from a patient are cultured in vitro, dispersed in a hydrogel, and then transplanted to a damaged tissue area. Because cells are injected directly, it is possible to regenerate a close-to-normal cartilage tissue. However, the conventional treatment techniques have problems. For example, conventional treatment techniques inevitably require performing a surgical procedure for obtaining the cells. In addition, conventional treatment techniques may include performing difficult in vitro culture processes, may be limited according to the number of cells depending on the size of the damaged tissue area, and may differentiate cells into cartilage tissue at a low rate in the body. One-time administration of the hydrogel-dispersed cells may temporarily reduce the pain and increase the mobility of the joint, but ultimately it is difficult to regenerate the damaged cartilage tissue at once with a single treatment.

In order to solve the problems in the prior art, the present inventors isolated only exosomes from stem cells differentiating into chondrocytes and found a cartilage regeneration effect of a composition containing the isolated exosomes, thereby completing the present invention.
(Patent Document 1) Korean Patent Publication No. 2013-0028012
(Patent Document 2) Korean Patent Registration No. 1279812
(Patent Document 3) Korean Patent Publication No. 2008-0082657
(Patent Document 4) Korean Patent Publication No. 2013-0072983
(Patent Document 5) Korean Patent Publication No. 2013-0009651

### SUMMARY OF INVENTION

One aspect of the present invention is to provide a composition for inducing chondrocyte differentiation or regenerating cartilage tissue including, as an active ingredient, exosomes derived from stem cells differentiating into chondrocytes.

Another aspect of the present invention is to provide a medium composition for inducing chondrocyte differentiation including the composition for inducing chondrocyte differentiation or regenerating cartilage tissue.

Also disclosed is an injection preparation for regenerating cartilage tissue including the composition for inducing chondrocyte differentiation or regenerating cartilage tissue.

Further another aspect of the present invention is to provide a pharmaceutical composition for treating cartilage disorders including the composition for inducing chondrocyte differentiation or regenerating cartilage tissue.

### DETAILED DESCRIPTION OF INVENTION

One embodiment of the present invention provides a composition for inducing chondrocyte differentiation or regenerating cartilage tissue including, as an active ingredient, exosomes derived from stem cells differentiating into chondrocytes.

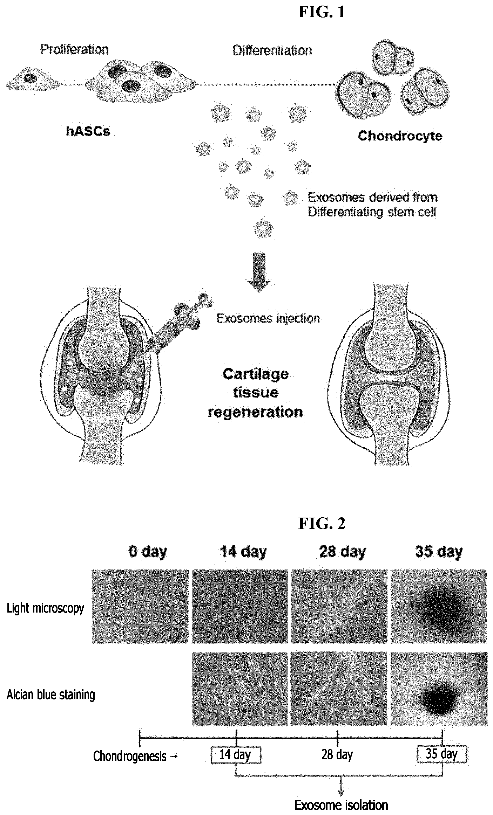

As used herein, the term "stem cells differentiating into chondrocytes" refers to stem cells that are currently differentiating into chondrocytes from stem cells, as shown in FIG. 1. From the stem cells differentiating into chondrocytes, it is possible to isolate exosomes containing any of chondrocyte genetic information, proteins, and growth factors.

When stem cells differentiate into chondrocytes, the shape and characteristics of the cells change. The exosomes can be isolated during differentiation at the time the shape and characteristics of the cells change, rather than from undifferentiated stem cells.

As used herein, the term "exosome" refers to a vesicle surrounded by a membrane, which can be secreted from various types of cells. An exosome can carry out various roles such as transferring materials from first cells and tissues to second cells and tissues by binding to the second cells and tissues. The materials transferred by an exosome may include any of membrane components, proteins, and ribonucleic acid (RNA).

Exosomes derived from the stem cells differentiating into chondrocytes may carry materials that define the basic characteristics of stem cells. For example, exosomes derived from the stem cells differentiating into chondrocytes may contain any of important growth factors, various bio-active proteins, and genetic information pertaining to cartilage cell differentiation.

The exosomes may be prepared by using an exosome isolation method known in the art. For example, the exosomes can be prepared by:
1) proliferating stem cells;
2) differentiating the proliferated stem cells into chondrocytes; and
3) isolating and purifying exosomes from the stem cells differentiating into chondrocytes.

The term "inducing chondrocyte differentiation" may refer to causing the differentiation of stem cells into chondrocytes.

The term "regenerating cartilage tissue" may refer to regenerating cartilage tissues by repairing damaged cartilage tissues, or by inducing the production of new cartilage tissues.

The stem cells differentiating into chondrocytes may be adult stem cells capable of differentiating into chondrocytes.

The adult stem cells capable of differentiating into chondrocytes may be bone marrow stem cells, umbilical cord blood stem cells, or adipose-derived stem cells. In specific embodiments, the adult stem cells may be adipose-derived stem cells.

Any of the bone marrow stem cells, the umbilical cord blood stem cells, and the adipose-derived stem cells may be stem cells derived from a human, an animal, or a plant.

The composition for cartilage regeneration according to the present invention includes exosomes derived from stem cells differentiating into chondrocytes, as an active ingredient for effectively regenerating cartilage. The exosomes derived from the stem cells differentiating into chondrocytes effectively induce cartilage regeneration, unlike compositions used in conventional techniques. The various growth factors associated with the proliferation and differentiation of cells, carried by the isolated and purified exosomes in the composition, causes the regeneration of cartilage tissue effectively and consistently. Thus, it is possible to solve the problems of the conventional methods discussed above, such as the defects resulted from in vitro cell culture of autologous chondrocytes or adult stem cells, the production of fibrocartilage, or the calcification of tissue due to apoptosis.

The stem cell-derived exosomes isolated during the differentiation of the stem cells into chondrocytes have the features of the stem cells, and can effectively deliver active factors associated with the differentiation of the stem cells into chondrocytes. The stem cell-derived exosomes thereby have the same benefits as previous treatments using stem cells, while minimizing the adverse effects of previous treatments utilizing stem cells.

The stem cell-derived exosomes isolated when the stem cells differentiate into chondrocytes, according to embodiments of the present invention, are bio-membrane vesicles secreted from cells. In addition, since the exosomes have a lipid structure similar to a cell membrane, they have an excellent absorption rate into peripheral cells when injected into the body. Thus, it is possible to induce effective regeneration of cartilage tissue by a rapid delivery of effective substances from the exosomes into a damaged tissue area.

Another embodiment of the present invention provides a medium composition for inducing chondrocyte differentiation. The medium includes the above-described composition for inducing chondrocyte differentiation or regenerating cartilage tissue.

The medium composition may contain the exosomes at a concentration of 1 to 100 µg/mL. In a specific embodiment, the medium composition contains the exosomes at a concentration of 1 to 60 µg/mL, for example, at a concentration of 50 µg/mL. However, the concentration is not limited thereto.

The medium composition for inducing chondrocyte differentiation may further include differentiation-inducing materials such as any of dexamethasone, insulin, ascorbate, IGF (Insulin-like Growth Factor) (a growth factor for chondrogenesis), and TGF-β1(Transforming Growth Factor β1), etc., which may cause the stem cells to differentiate into chondrocytes, but embodiments are not limited thereto.

Still another embodiment of the present invention provides an injection preparation for regenerating cartilage tissue. The injection preparation may include the composition for inducing chondrocyte differentiation or regenerating cartilage tissue.

The injection preparation may further include phosphate-buffered saline (PBS). That is, the injection preparation may include the composition for inducing chondrocyte differentiation or regenerating cartilage tissue contained in a solution of PBS.

The injection preparation may include a hydrogel instead of PBS.

The hydrogel may include any one of hyaluronic acid, gelatin, alginate, chitosan, fibrin, elastin, collagen, and methylcellulose. In a specific embodiment, the hydrogel may be a hydrogel of hyaluronic acid, but embodiments are not limited thereto.

The injection preparation may contain exosomes at a concentration of 10 to 1000 µg/mL, specifically at a concentration of 10 to 900 µg/mL, more specifically at a concentration of 10 to 800 µg/mL. For example, the injection preparations may include exosomes at a concentration of 500 µg/mL, but the injection preparation is not limited thereto.

The injection preparation may be administered to mammals such as rats, mice, livestock, or humans, by injecting the injection preparation into an area including damaged cartilage, or the like.

The composition for cartilage regeneration according to embodiments of the present invention can be applied to a patient relatively quickly and at a low cost, because the composition is easily injected into the body in the form of an injection preparation. The composition according to embodiments of the present invention reduces the suffering, sequelae, and economic burden of a patient who would otherwise be treated using conventional methods. In addition, the exosomes isolated during the differentiation of stem cells into chondrocytes contain materials for inducing extracellular matrix, and various growth factors associated with cell proliferation and differentiation. The exosomes thereby effectively induce regeneration in damaged cartilage tissues. Accordingly, a long-term effect can be expected with a one-time treatment using the composition. The composition may be used without conventional problems. For example, the composition does not need to be applied in multiple, periodic treatments in order to cause a sustaining effect.

Another embodiment of the present invention includes a pharmaceutical composition for treating cartilage disorders including the composition for inducing chondrocyte differentiation or regenerating cartilage tissue.

As used herein, the term "cartilage disorder" may be a cartilage disorder resulting from damage to cartilage tissues. In a specific example, a cartilage disorder may be selected from the group consisting of osteoarthritis, osteoarthrosis, dyschondroplasia, degenerative arthritis, rheumatoid arthritis, osteomalacia, fibrous osteitis, and aplastic bone disease, but is not limited thereto.

As used herein, the term "treatment of cartilage disorder" may refer to treating damage to cartilage tissue by injecting a composition for treating cartilage disorders via an intra-articular route to regenerate the damaged cartilage.

The pharmaceutical composition according to the above embodiment may include various oral or parenteral formulations. The formulations may be prepared using a diluting agent or an excipient, such as commonly-used fillers, weighting agents, binding agents, wetting agents, disintegrating agents, surfactants, and the like.

Solid formulations for oral administration of the pharmaceutical composition may include tablets, pills, powders, granules, capsules and the like. Such solid formulations may be prepared by mixing at least one compound with one or more excipients, for example, one or more of a starch, calcium carbonate, sucrose or lactose, gelatin, and the like. Further, in addition to simple excipients, solid formulations may include lubricants such as magnesium stearate, talc, and the like.

Liquid formulations for oral administration of the pharmaceutical composition may include a suspension, a liquid for internal use, an emulsion, a syrup, and the like. In addition to commonly used simple diluents such as water and liquid paraffin, the liquid formulations for oral administration may also include various excipients, for example, wetting agents, sweetening agents, flavoring agents, preservatives, and the like.

Formulations for parenteral administration of the pharmaceutical composition may include any of sterilized aqueous solutions, non-aqueous solvents, suspensions, emulsions, lyophilization formulations, and suppositories.

Pharmaceutical compositions according to embodiments of the present invention may include non-aqueous solvents and suspending agents. For example, the pharmaceutical compositions may include vegetable oils, such as any of propylene glycol, polyethylene glycol, and olive oil. According to embodiments, the pharmaceutical compositions may include an injectable ester, such as ethyl oleate, and the like. Suppositories containing the pharmaceutical compositions may also include WITEPSOL, macrogol, tween 61, cacao butter, laurinum, glycerol, gelatin, and the like.

The dosage forms of the pharmaceutical composition according to the above embodiments may be in the form of a pharmaceutically acceptable salt of the exosome compound, or the exosome compound may be used alone or in suitable combination with other pharmaceutically active compounds. The salt of the exosome compound is not particularly limited as long as it is pharmaceutically acceptable, and may include, for example, hydrochloric acid, sulfuric acid, nitric acid, phosphoric acid, hydrofluoric acid, hydrobromic acid, formic acid, acetic acid, tartaric acid, lactic acid, citric acid, fumaric acid, malic acid, succinic acid, methanesulfonic acid, benzenesulfonic acid, toluenesulfonic acid, naphthalenesulfonic acid and the like.

The pharmaceutical composition according an embodiment may be parenterally or orally administered depending on the intended use. The daily dosage may be 0.1 to 500 mg per 1 kg of body weight. For example, the daily dosage may be 1 to 100 mg per 1 kg of body weight. The administration frequency may be once a day or a few times a day. The effective dosage for a specific patient may vary depending on the patient's body weight, age, gender, health condition, diet excretion rate, severity of disease, and the like, as well as the desired administration time and administration method.

The pharmaceutical compositions according to the above embodiments may be formulated into any form suitable for a pharmaceutical preparation, including oral preparations such as powders, granules, tablets, capsules, suspensions, emulsions, syrups, aerosols and the like, external preparations such as ointments, creams and the like; suppositories; and sterilized injectable preparation solutions in accordance with conventional methods.

The pharmaceutical compositions according to embodiments may be administered to mammals such as rats, mice, livestock, humans, and the like, using various routes such as any of parenteral routes, oral routes, and the like. Although all routes of administration can be expected, it may be preferably administered orally; rectally; or by intravenous, intramuscular, subcutaneous, intrauterine, or intracerebroventricular injections.

The pharmaceutical composition according to embodiments may further include differentiation-inducing materials such as dexamethasone, insulin, ascorbate, IGF(Insulin-like Growth Factor), which is a growth factor for chondrogenesis, and TGF-β1 (Transforming Growth Factor β1), etc., in order to differentiate the stem cells into chondrocytes, but is not limited thereto.

Still further embodiments of the present invention include a method for treating cartilage disorders including administering, to a mammal, a therapeutically effective amount of the composition for inducing chondrocyte differentiation or regenerating cartilage tissue.

The administered composition may be a pharmaceutical composition for treating cartilage disorders, which contains the composition for inducing chondrocyte differentiation or regenerating cartilage tissue. The administered composition may be an injection preparation for cartilage regeneration including the composition for inducing chondrocyte differentiation or regenerating cartilage tissue. Details regarding the pharmaceutical composition for treating cartilage disorders or the injection preparation for cartilage generation may be similarly applicable to the treatment method.

The composition may be administered to mammals suffering from cartilage disorders. For example, the mammals may be any of rats, mice, livestock, humans, and the like, which suffer from cartilage disorders.

The term "therapeutically effective amount" refers to a sufficient amount of the composition to provide a therapeutic response for treating cartilage disorders in mammals.

The dosage form may be via parental administration or oral administration, or, for example, the method may include administering the composition by injecting the composition into a damaged area, such as a site of damaged cartilage of a mammal.

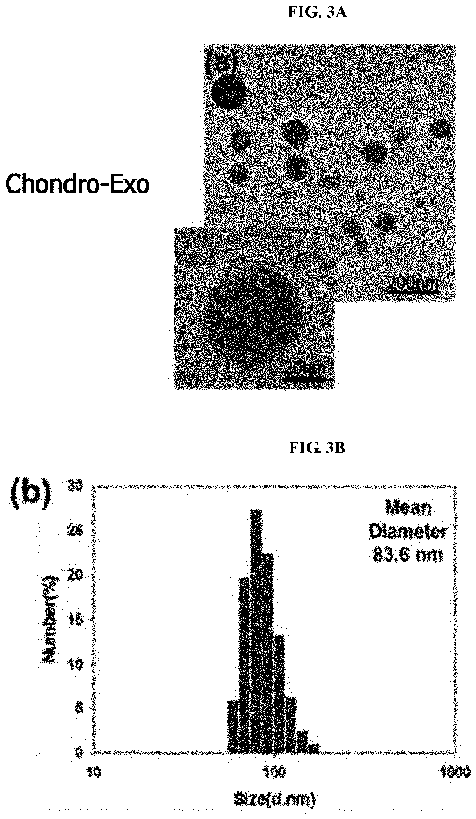

In one embodiment of the present invention, the size of the exosomes derived from proliferating stem cells (ASC-EXO) and the exosomes derived from stem cells differentiating into chondrocytes (Chondro-EXO) was confirmed. As a result, the average diameter of the exosomes of ASC-EXO was about 88.17 nm, and the average diameter of the exosomes of Chondro-EXO was about 83.6 nm(see FIG. 3A-3D).

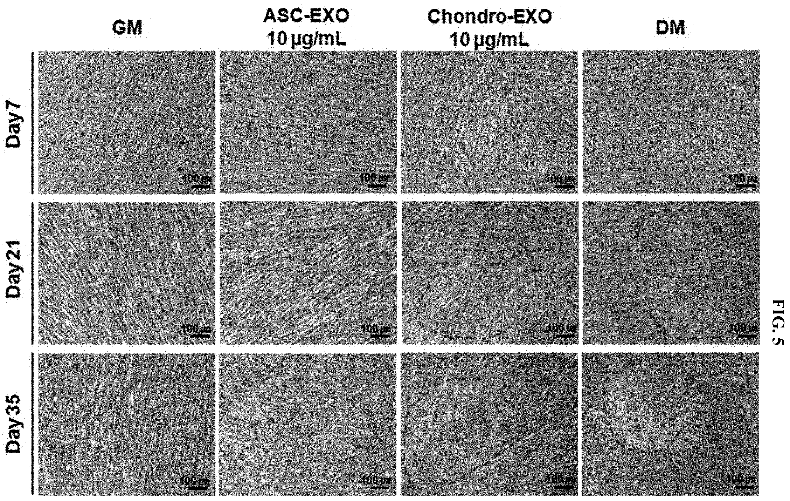

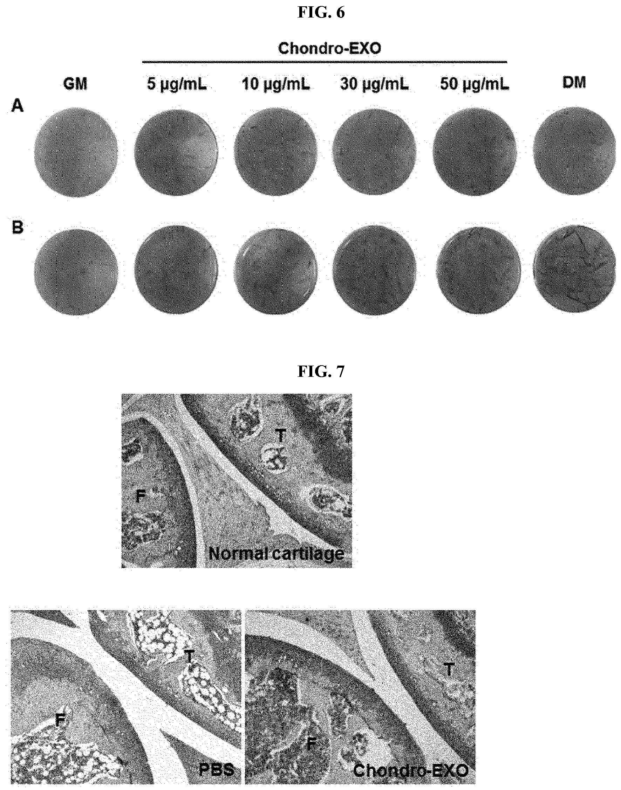

In another embodiment of the present invention, when a medium composition, which contains the exosomes derived from stem cells differentiating into chondrocytes (Chondro-EXO), was applied to stem cells for chondrogenesis, precartilage condensation appeared at a similar level to that of the positive control after day 21 of the treatment with the exosomes, and a cartilage-specific matrix was observed. However, the precartilage condensation and cartilage-specific matrix were not observed when the exosomes derived from proliferating stem cells (ASC-EXO) or the negative control was applied to stem cells for chondrogenesis. When treated with the ASC-EXO or the negative control, only proliferation of stem cells was observed (See FIGS. 5 and 6).

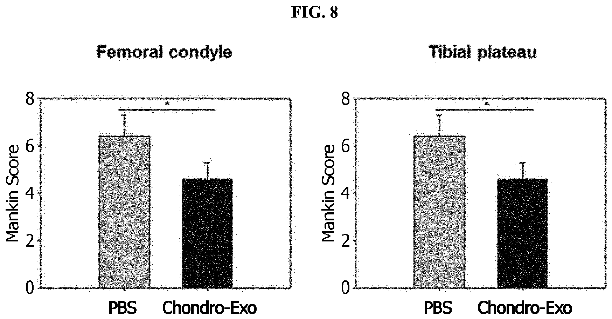

In an embodiment of the present invention, the cartilage tissue effectively regenerated when the composition containing the exosomes derived from the stem cells differentiating into chondrocytes (Chondro-EXO) was injected into the body (e.g., as shown in FIGS. 7 and 8).

### ADVANTAGEOUS EFFECTS

The composition for inducing chondrocyte differentiation or cartilage regeneration according to a specific embodiment of the present invention includes, as an active factor, exosomes derived from stem cells differentiating into chondrocytes. The exosomes, which are secreted during stem cell differentiation, contain growth factors associated with chondrocyte differentiation, as well as genes and proteins associated with stem cell proliferation and regeneration. Thus, the exosomes can be used to induce cartilage tissue regeneration. In addition, the composition can stably and rapidly deliver active materials, using the exosomes as cell-derived vehicles, into cells, while having fewer side effects than a conventional cell therapeutic agent. Therefore, a patient's pain can be reduced through a simple operation using an injectable composition for preventing and treating cartilage disorders, and the cartilage disorders can be continuously and effectively treated after the operation.

### BRIEF DESCRIPTION OF DRAWINGS

FIG. 1 is a schematic diagram of exosomes derived from stem cells differentiating into chondrocytes, and the application of the exosomes, according to an embodiment.
FIG. 2 shows times when exosomes are isolated from stem cells differentiating into chondrocytes according to an embodiment. FIG. 2 shows changes in the shape of stem cells differentiating into chondrocytes, and the synthesis of a cartilage-specific matrix confirmed by alcian blue staining.
FIG. 3A illustrates transmission electron microscope images showing the structure and shape of exosomes derived from stem cells differentiating into chondrocytes (Chondro-Exo) according to an embodiment.
FIG. 3B is a graph showing a distribution of diameters of exosomes in a Chondro-Exo sample obtained using a nanoparticle analyzer and dynamic light scattering according to an embodiment.
FIG. 3C illustrates transmission electron microscope images showing the structure and shape of exosomes derived from proliferating stem cells (ASC-Exo).
FIG. 3D is a graph showing a distribution of diameters of exosomes in an ASC-Exo sample obtained using a nanoparticle analyzer and dynamic light scattering according to an embodiment.
FIG. 4 shows images obtained using Exo-Check™ exosome antibody arrays of membrane surface markers of exosomes derived from stem cells differentiating into chondrocytes according to an embodiment.
FIG. 5 shows results of inducing differentiation of human adipose-derived stem cells into chondrocytes to an embodiment: the label "GM" indicates a growth medium for stem cells; the label "ASC-EXO" indicates exosomes derived from proliferating stem cells; the label "Chondro-EXO" indicates exosomes derived from stem cells differentiating into chondrocytes; the label "DM" indicates a differentiation medium into chondrocytes; and a dotted line indicates an area where precartilage condensation phenomenon occurs.
FIG. 6 shows an analysis result after 21 days of induction of differentiation of human adipose-derived stem cells into chondrocytes according to an embodiment. In FIG. 6, the label "A" indicates the synthesis of acidic mucopolysaccharides, for example, an acidic mucosubstance and acidic mucin. The acidic mucopolysaccharides observed in FIG. 6 are part of cartilage-specific matrices, as confirmed through alcian blue staining. The label "B" indicates the synthesis of proteoglycan, which is a cartilage-specific matrix, confirmed through safranin-o staining. The label "GM" indicates a growth medium for stem cells. The label "Chondro-EXO" refers to exosomes derived from stem cells differentiating into chondrocytes. The label "DM" refers to a differentiation medium into chondrocytes.
FIG. 7 shows a 100x microscopic images of safranin-o-stained joint cavities of a mouse having normal cartilage, a mouse having osteoarthritis injected with phosphate-buffered saline (PBS), and a mouse having osteoarthritis injected with exosomes derived from stem cells differentiating into chondrocytes according to an embodiment. Specifically, FIG. 7 confirms the degree of regeneration of cartilage tissue after injecting exosomes derived from stem cells differentiating into chondrocytes (Chondro-EXO), as compared to the normal cartalige and PBS control groups. In FIG. 7, the label "T" indicates the mouse tibia, and the label "F" indicates the mouse femur.
FIG. 8 illustrates graphs showing Mankin scores indicating the degree of induction of osteoarthritis in a femoral condyle and a tibial plateau, based on table 1. The label "PBS" refers to a model injected with PBS as a negative control group, and the label "Chondro-EXO" refers to a model injected with exosomes derived from stems cells differentiating into chondrocytes.

### EXAMPLES

Hereinafter, the present invention will be described with reference to non-limiting examples. It is to be understood, however, that these examples are for illustrative purposes only and are not intended to limit the scope of the present invention.

### <Example 1> Isolation of exosomes from stem cells differentiating into chondrocytes

In order to isolate exosomes from the stem cells differentiating into chondrocytes, human adipose-derived stem cells were grown to 80 to 90% confluency in a normal growth medium, for example, Dulbecco Modified Eagle Medium high glucose (DMEM) containing 10% by weight fetal bovine serum and 1% by weight penicillin/streptomycin. Then, the medium was replaced with a differentiation medium, for example, Dulbecco Modified Eagle Medium high glucose (DMEM) containing 5% by weight of fetal bovine serum, 1% by weight of penicillin/streptomycin, 100 nM dexamethasone, 0.15 mM ascorbic acid, 1X ITS (Insulin-Transferrin-Sodium selenite), and 10 ng/mL TGF-β1(Transforming Growth Factor β1). The human adipose-derived stem cells were cultured for a total of 5 weeks, in order to differentiate the human adipose-derived stem cells into chondrocytes.

After replacing the normal growth medium with the differentiation medium, the stem cells were placed in a serum-free medium and phenol red-free DMEM medium before isolating the exosomes, and maintained therein for 24 hours.

Then, a cell culture supernatant was recovered. The recovered cell culture supernatant was centrifuged at 300 xg for 10 minutes to remove the cells, and centrifuged at 2,000 xg for 30 minutes to remove the cell secretions. Thereafter, the cells were concentrated by centrifugation at 5000xg for 60 minutes using a centrifuge tube (molecular weight cut off = 3000, amicon tube) equipped with a filter having a molecular weight of 3000. The supernatant obtained after the concentration step was mixed with an exosome isolation reagent at a ratio of 1:0.5 by weight and stored at 4° C for one day.

Subsequently, the cells were centrifuged at 10000xg for 60 minutes to obtain an exosome precipitate, which was then filtered through a 0.22 µm filter, and washed with phosphate-buffered saline (PBS). Specifically, the exosome precipitate was filtered through an exosome spin column. The washed exosome precipitate was centrifuged at 10000xg for 60 minutes and then resuspended in PBS.

After recovering the supernatant, the differentiation medium was added again to induce chondrocyte differentiation of the stem cells. The chondrocyte differentiation of the stem cells, the recovery of the supernatant and the exosome isolation from the supernatant were repeated until week 5.

As shown in FIG. 2, 14 days after the differentiation media was added, precartilage condensation began to be observed during the differentiation of stem cells into chondrocytes. After 35 days, a formation of colonies of differentiated chondrocytes was confirmed.

Accordingly, exosomes were isolated from the supernatant recovered from 14 days (2 weeks) to 35 days (5 weeks) after the induction of differentiation, in which the change in cell shape was clearly observed.

### <Comparative Example 1> Isolation of exosomes from proliferating stem cells

In order to compare the efficacy of the exosomes derived from the stem cells differentiating into chondrocytes, exosomes were isolated from proliferating human adipose-derived stem cells and used as a comparative group.

Specifically, the exosomes (ASC-EXO) were isolated from the proliferating human adipose-derived stem cells in the same manner as described with reference to Example 1, except that the differentiation medium was not used.

### <Example 2> Microscopic analysis of exosomes

The size and shape of the exosomes isolated from the stem cells differentiating into chondrocytes of Example 1 and the exosomes isolated from the proliferating stem cells were confirmed via a transmission electron microscope and a nanoparticle analyzer using dynamic light scattering. The exosome membrane surface proteins were confirmed using Exo-Check™ exosome antibody arrays, which indicate the presence or absence of specific protein expression.

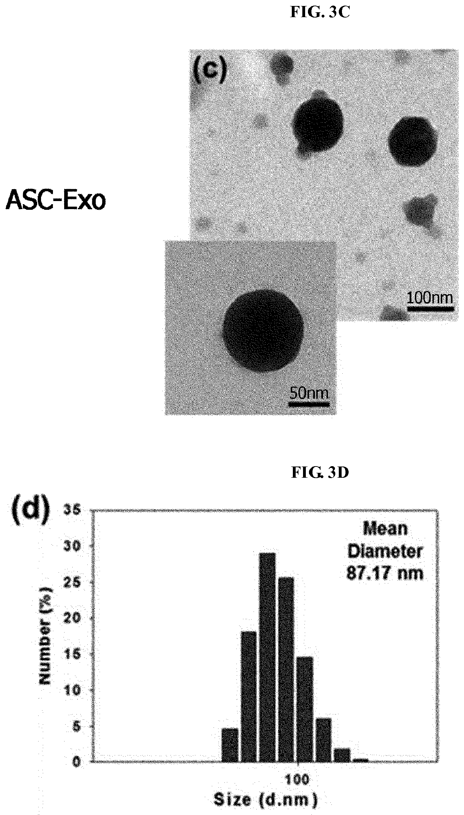

As shown in FIGS. 3A and 3C, the shape of the isolated exosomes could be confirmed by a transmission electron microscope. In addition, as shown in FIGS. 3B and 3D, the diameters of the exosomes were confirmed to be about 83.6 nm (Chondro-EXO) and 87.17 nm (ASC-EXO) on average.

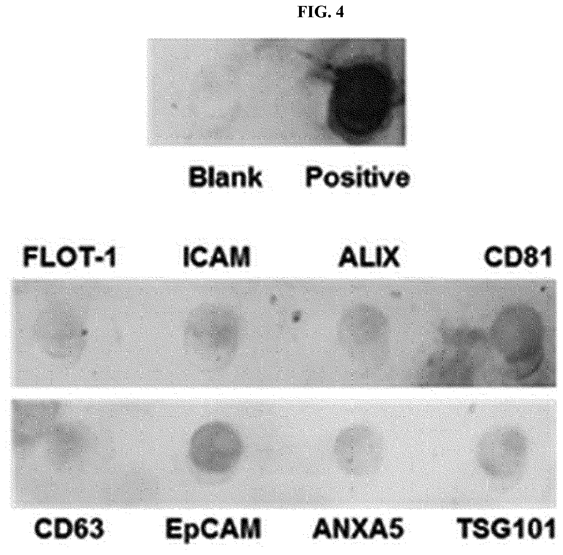

As shown in FIG. 4, the expression of exosome-specific markers (such as CD63, CD81, ALIX, FLOT1, ICAM1, EpCam, ANXA5 and TSG101), which are known as exosome membrane surface markers, was confirmed through an antibody reaction using the exosome antibody arrays.

### <Example 3> Induction of chondrocyte differentiation using exosomes

In order to induce chondrocyte differentiation of human adipose-derived stem cells using exosomes, a medium composition containing exosomes derived from proliferating stem cells (ACS-EXO), and a medium composition containing exosomes derived from the stem cells differentiating into chondrocytes(Chondro-EXO), were used. The medium composition was used by adding the exosomes at a concentration of 10 µg/mL to a growth medium for stem cells, for example, Dulbecco Modified Eagle Medium high glucose (DMEM) containing 5% by weight fetal bovine serum and 1% by weight penicillin/streptomycin.

As a negative control group (indicated by growth medium (GM) in FIG. 5), the growth medium for stem cells, including Dulbecco Modified Eagle Medium high glucose(DMEM) containing 5% by weight fetal bovine serum and 1% by weight penicillin/streptomycin, was used. As a positive control group (indicated as differentiation medium (DM) in FIG. 5), the differentiation medium into chondrocyte, including Dulbecco Modified Eagle Medium high glucose (DMEM) containing 5% by weight fetal bovine serum, 1% by weight penicillin/streptomycin, 100 nM dexamethasone, 0.15 mM ascorbic acid, 1X ITS (Insulin-Transferrin-Sodium selenite) and 10 ng/mL TGF-β1(Transforming Growth Factor β1), was used.

The medium composition was replaced every 3 days for 35 days, and the change in cell shape for the stem cells in which the differentiation into chondrocytes was induced was confirmed using a microscope.

As shown in FIG. 5, precartilage condensation appeared in the cells treated with the Chondro-EXO at a similar level to the positive control, after 21 days of induction of differentiation. In addition, it was confirmed that the cells in the negative control group (GM) and the group treated with the exosomes (ASC-EXO) proliferated without the occurrence of precartilage condensation.

### <Example 4> Analysis of differentiation ability of chondrocytes using exosomes

In order to confirm the induction of chondrocyte differentiation using the exosomes, a medium composition containing the exosomes derived from the stem cells differentiating into chondrocytes (Chondro-EXO) was used. The medium composition was used by adding the exosomes derived from the stem cells differentiating into chondrocytes at concentrations of 5, 10, 30, 50 µg/mL to a growth medium for stem cells. The growth medium included Dulbecco Modified Eagle Medium high glucose (DMEM) containing 5% by weight fetal bovine serum and 1% by weight penicillin/streptomycin.

As a negative control group, the growth medium for stem cells, including Dulbecco Modified Eagle Medium high glucose (DMEM) containing 5% by weight fetal bovine serum and 1% by weight penicillin/streptomycin, was used. As a positive control group, a differentiation medium for inducing differentiation into chondrocytes, including Dulbecco Modified Eagle Medium high glucose (DMEM) containing 5% by weight fetal bovine serum, 1% by weight penicillin/streptomycin, 100 nM dexamethasone, 0.15 mM ascorbic acid, 1X ITS (Insulin-Transferrin-Sodium selenite) and 10 ng/mL TGF-β1(Transforming Growth Factor β1) was used.

For each sample, the medium composition was replaced once every 3 days for 21 days, and the differentiation of cells was analyzed by alcian blue staining and safranin-o staining.

As shown in FIG. 6, a chondrocyte-specific extracellular matrix was formed when the exosomes derived from the stem cells differentiating into chondrocytes (Chondro-EXO) were treated. As shown in the samples labeled "A," alcian blue stains acidic mucopolysaccharides (e.g., an acidic mucosubstance and acidic mucin) in the cartilage-specific matrix blue. As shown in the samples labeled "B," safranin-o stains proteoglycan in the cartilage-specific matrix red.

In addition, each staining confirms that a cartilage-specific matrix is formed when the exosomes are applied at different concentrations to the stem cells. Even in the medium containing the exosomes at a low concentration (5 µg/mL), the differentiation of the stem cells into chondrocytes was confirmed.

Accordingly, it was confirmed that the exosomes derived from the stem cells differentiating into chondrocytes showed an excellent effect of inducing differentiation into chondrocytes from stem cells.

### <Example 5> Evaluation of cartilage regeneration in vivo using exosomes derived from stem cells differentiating into chondrocytes

The substantial in vivo cartilage tissue regenerating effect of the exosomes derived from the stem cells differentiating into chondrocytes was confirmed using a DMM (Destabilization of the medial meniscus) arthritis-inducing mouse model, which is a commonly used model of osteoarthritis.

In particular, after cleanly removing hair around the knee of each mouse, the knee joint was incised about 1 cm along the side of the patella using a surgical tool. After exposing the part of the joint capsule and incising it, the medial meniscotibial ligament connected to the medial meniscus was cut, the joint capsule and skin were closed in sequence, and finished with a suture.

The composition containing the exosomes was injected into the joint cavity once a week, from week 5 to week 10, during which time osteoarthritis progressed after the operation. The exosome composition was prepared by carrying the exosomes derived from the stem cells differentiating into chondrocytes of Example 1 in phosphate-buffered saline (PBS). The final concentration of the exosomes in the exosome composition was 500 µg/mL. 6 µL of the composition (3 µg/6 µL per mouse) was injected into the joint cavity of each of the mice. Phosphate-buffered saline (PBS) was used as a negative control. After 11 weeks, the regeneration of the cartilage tissue was confirmed using safranin-o staining.

As shown in FIG. 7, it was confirmed that, when applying the composition containing the exosomes derived from the stem cells differentiating into chondrocytes, the cartilage-specific matrix was synthesized in a relatively large amount compared with the negative control, and the cartilage was regenerated similarly to natural cartilage without damaging the cartilage surface (superficial zone). The regenerated cartilage was stained red. Therefore, it was confirmed that the composition containing the exosomes derived from the stem cells differentiating into chondrocytes had an excellent cartilage tissue regenerating effect.

In addition, an evaluation using the Mankin score, which is a basic histopathological observation for evaluating osteoarthritis, was performed on the basis of the surface damage of cartilage induced by osteoarthritis, chondrocyte stainability, the change of a tide mark which is the boundary between the cartilage and bone, and graded with scores. The higher score refers to the higher degree of osteoarthritis induction, that is, the higher degree of cartilage damage and the results of the Mankin scores are shown in Tables 1 and 2 below.

**[Table 1] Femoral condyle**

| Group | Mankin Scores (Max=14) | | | | |
|---|---|---|---|---|---|
| | Cartilage structure | Chondrocytes | Safranin-O staining | Tidemark | Sum |
| PBS | 2.80±0.37 | 2.20±0.2 | 2.00±0.45 | 0 | 7.00±0.89 |
| Chondro-EXO | 1.80±0.37 | 0.80±0.2 | 1.80±0.58 | 0 | 4.60±0.87 |

**[Table 2] Tibial plateau**

| Group | Mankin Scores (Max=14) | | | | |
|---|---|---|---|---|---|
| | Cartilage structure | Chondrocytes | Safranin-O staining | Tidemark | Sum |
| PBS | 2.60±0.24 | 1.80±0.2 | 1.80±0.37 | 0 | 6.40±0.89 |
| Chondro-EXO | 2.00±0.32 | 1.20±0.2 | 1.40±0.40 | 0 | 4.60±0.68 |

As shown in FIG. 8 and the results of Tables above, when the composition containing the exosomes derived from the stem cells differentiating into chondrocytes was injected, the surface damage and hypercellularity of cartilage is low, and the synthesis of cartilage-specific matrix was judged to be high, and thus a decrease in the Mankin score was acknowledged (Table 1, Table 2 and FIG. 8).

## Claims

1. A composition, suitable for inducing chondrocyte differentiation for regenerating cartilage tissue, comprising, as an active ingredient, exosomes derived from human adipose-derived stem cells differentiating into chondrocytes.

2. The composition of Claim 1 for use in a therapeutic method.

3. Use of a culture medium for inducing chondrocyte differentiation *in vitro* comprising the composition according to Claim 1.

4. The use of Claim 3, wherein the culture medium contains the exosomes at a concentration of 1 to 100 µg/ml.

5. A pharmaceutical composition for use in a method of treating cartilage disorders comprising the composition according to Claim 1 or 2.

## Patentansprüche

1. Zur Induktion der Chondrozyten-Differenzierung geeignete Zusammensetzung zum Regenerieren von Knorpelgewebe, als aktiven Inhaltsstoff von humanen, in Chondrozyten differenzierenden, Fett-abgeleiteten Stammzellen abgeleitete Exosomen enthaltend.

2. Zusammensetzung nach Anspruch 1 zur Anwendung in einem therapeutischen Verfahren.

3. Verwendung eines Kulturmediums, welches die Zusammensetzung gemäß Anspruch 1 beinhaltet, zum Induzieren von Chondrozyten-Differenzierung *in vitro.*

4. Verwendung nach Anspruch 3, wobei das Kulturmedium die Exosomen in einer Konzentration von 1 bis 100 µg/ml beinhaltet.

5. Pharmazeutische Zusammensetzung zur Anwendung in einem Verfahren der Behandlung von Knorpel-Leiden, wobei die pharmazeutische Zusammensetzung die Zusammensetzung gemäß Anspruch 1 oder 2 beinhaltet.

## Revendications

1. A une composition
capable d'induire la différenciation des chondrocytes pour la régénération du tissu cartilagineux,
comprenant, comme ingrédient actif, des exosomes dérivés de cellules souches humaines, provenant de tissus adipeux, qui se différenciant en chondrocytes.

2. La composition de la revendication 1 pour l'utilisation dans une méthode thérapeutique.

3. Utilisation d'un milieu de culture pour induire la différenciation des chondrocytes in vitro comprenant la composition selon la revendication 1.

4. L'utilisation de la revendication 3, dans laquelle le milieu de culture contient les exosomes à une concentration de 1 à 100 µg/ml.

5. Composition pharmaceutique à utiliser dans une méthode de traitement des désordres du cartilage comprenant la composition selon la revendication 1 ou 2.
